(19) Europäisches Patentamt
European Patent Office
Office européen des brevets

(11) Veröffentlichungsnummer: **0 008 345**
**A1**

(12) # EUROPÄISCHE PATENTANMELDUNG

(21) Anmeldenummer: **79102316.1**

(22) Anmeldetag: **09.07.79**

(51) Int. Cl.³: **C 07 D 239/10**
//D06M1/00

(30) Priorität: **14.07.78 DE 2830997**

(43) Veröffentlichungstag der Anmeldung:
**05.03.80 Patentblatt 80/5**

(84) Benannte Vertragsstaaten:
**BE CH DE FR GB IT**

(71) Anmelder: **BASF Aktiengesellschaft**
**Carl-Bosch-Strasse 38**
**D-6700 Ludwigshafen(DE)**

(72) Erfinder: **Petersen, Harro, Dr. Chem.**
**Kalmitstrasse 23**
**D-6710 Frankenthal(DE)**

(54) **Hexahydropyrimidyl-4-äther und ein Verfahren zur Herstellung von Hexahydropyrimidyl-4-äthern.**

(57) Neue Hexahydropyrimidyl-4-äther und ein Verfahren zur Herstellung von Hexahydropyrimidyl-4-äthern durch Umsetzung von Hexahydropyrimidinen mit Alkoholen bei Temperaturen über 110°C.

Die nach dem Verfahren der Erfindung herstellbaren Hexahydro-pyrimidyl- (4)- äther sind wertvolle Ausgangsstoffe für die Herstellung von Farbstoffen, Schädlingsbekämpfungsmitteln und Hilfsmitteln in der Textilchemie, Lackharzchemie, Holzwerkstoffindustrie.

EP 0 008 345 A1

BASF Aktiengesellschaft O. Z. 0050/033272

# Hexahydropyrimidyl-4-äther und ein Verfahren zur Herstellung von Hexahydropyrimidyl-4-äthern

Die Erfindung betrifft neue Hexahydropyrimidyl-4-äther und ein Verfahren zur Herstellung von Hexahydropyrimidyl-4-äthern durch Umsetzung von Hexahydropyrimidinen mit Alkoholen bei Temperaturen über 110°C.

Für die Herstellung von 2-Oxo(thiono)-hexahydropyrimidyl-4-äthern ist ein Verfahren bekannt, bei dem Harnstoffe mit Aldehyden in Gegenwart einer Säure bei Temperaturen unterhalb 110°C umgesetzt werden (Synthesis 1973, Seiten 262 und 263; DT-PS 1 231 247). Nach den Angaben muß mit einem hohen Überschuß der Alkohole umgesetzt werden, um für die technischen Verwendungsgebiete befriedigende Ausbeuten zu erhalten. Setzt man säureempfindliche Alkohole, z.B. oxäthylierte Carbonsäuren, ungesättigte Carbonsäuren mit Hydroxylgruppen, Polyalkohole, Polyätherdiole, ein, liefert dieses Herstellungsverfahren keine wesentliche Menge an Endstoff. Die Neutralisation der Säure und Abtrennung der dabei entstehenden Salze sind weitere Erschwernisse bei der Aufarbeitung.

Die deutsche Offenlegungsschrift 17 70 089 lehrt ein Verfahren zur Herstellung von fluorierten 4-Alkoxy-propylenharnstoffen durch Umsetzung von 4-Alkoxy(Hydroxy)-propylenharnstoffen mit fluorierten aliphatischen Alkoholen in Ge-

genwart einer nicht oxidierenden Säure. Es wird angegeben, daß die Umsetzung in der Regel bei einer Temperatur zwischen 20 und 100°C, vorzugsweise zwischen 40 und 80°C, durchgeführt wird.

Es wurde nun gefunden, daß man Hexahydropyrimidyl-(4)-äther der Formel

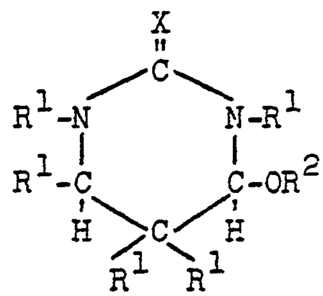

I,

worin die einzelnen Reste $R^1$ und $R^2$ gleich oder verschieden sein können und jeweils einen cycloaliphatischen oder araliphatischen Rest bedeuten, darüber hinaus auch die Reste $R^1$ jeweils ein Wasserstoffatom, einen aromatischen oder einen aliphatischen Rest bezeichnen können, $R^2$ auch für einen aliphatischen Rest, der darüber hinaus n Reste

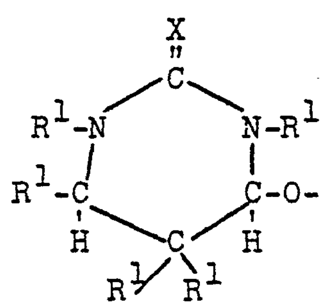

enthalten kann, stehen kann, n eine ganze Zahl von 1 bis 5 bezeichnet, X ein Sauerstoffatom oder ein Schwefelatom bedeutet, durch Umsetzung von 4-Hydroxy-hexahydropyrimidinen oder seinen Äthern mit Alkoholen vorteilhaft erhält, wenn man Hexahydropyrimidine der Formel

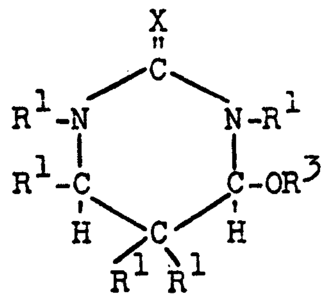

II,

worin $R^1$ und X die vorgenannte Bedeutung besitzen und $R^3$ ein Wasserstoffatom bezeichnet, darüber hinaus auch, wenn $R^2$ für einen aliphatischen Rest mit mindestens 5 Kohlenstoffatomen oder einen cycloaliphatischen oder araliphatischen Rest steht, auch eine Alkylgruppe mit 1 bis 3 Kohlenstoffatomen bedeuten kann, mit Alkoholen der Formel

$$R^4\text{-OH} \qquad \text{III},$$

worin $R^4$ die vorgenannte Bedeutung von $R^2$ besitzt, oder, wenn $R^2$ für einen aliphatischen Rest mit n Resten

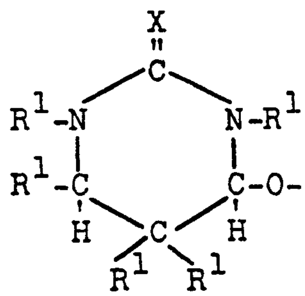

steht, auch denselben aliphatischen Rest mit anstelle der n Reste

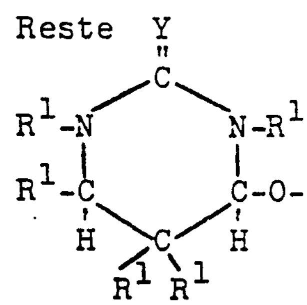

n Resten -OH bezeichnen kann, bei einer Temperatur oberhalb von 110°C umsetzt.

Weiterhin wurden die neuen Polyhexahydropyrimidyl-(4)-äther der Formel

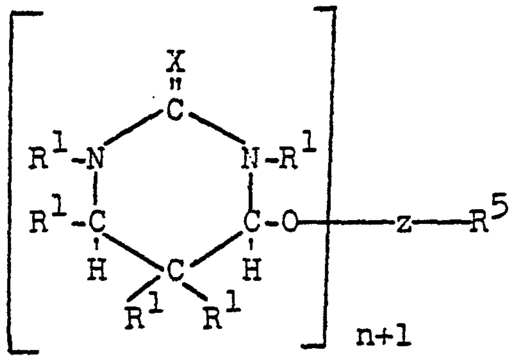 Ia,

worin die einzelnen Reste $R^1$ gleich oder verschieden sein können und jeweils ein Wasserstoffatom, einen aliphatischen, cycloaliphatischen, araliphatischen oder aromatischen Rest bedeuten, X ein Sauerstoffatom oder ein Schwefelatom bedeutet, n eine ganze Zahl von 1 bis 5 bedeutet, z eine n + 1 entsprechende Zahl an Einfachbindungen bezeichnet, $R^5$ für einen aliphatischen Rest steht, gefunden.

Die Umsetzung kann für den Fall der Verwendung von Methylglykol und von 2-Oxo-4-hydroxy-5,5-dimethyl-6-isopropylhexahydropyrimidin durch die folgenden Formeln wiedergegeben werden:

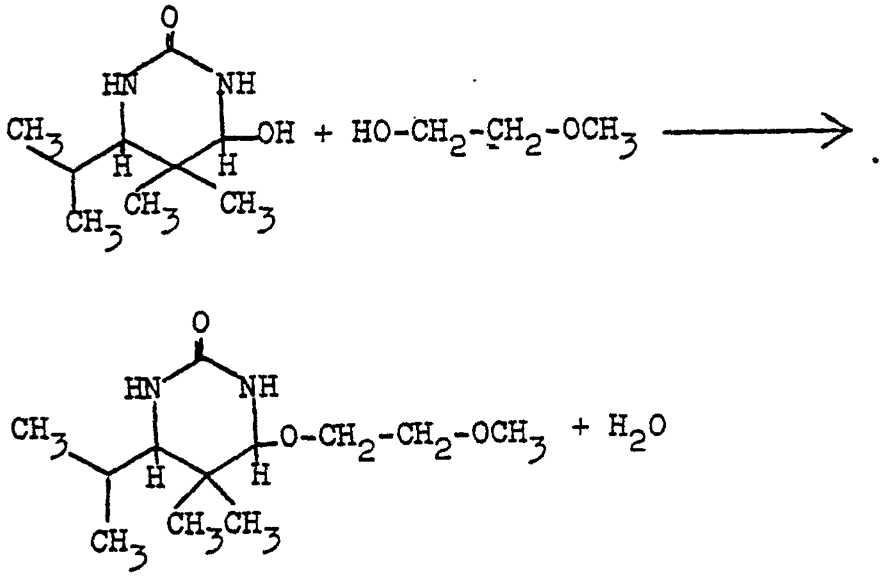

Im Hinblick auf den Stand der Technik liefert das Verfahren nach der Erfindung auf einfachem und wirtschaftlichem Wege eine große Zahl von bekannten und bisher nicht beschriebenen Hexahydropyrimidyl-4-äthern in besserer Ausbeute und Reinheit. Man braucht nicht in Gegenwart von Säure umzusetzen, die Neutralisation und die Abtrennung von Salzen entfallen. Zersetzungsreaktionen und Nebenreaktionen der Ausgangs- und Endprodukte werden vermieden. Ein Überschuß der Alkoholkomponente ist nicht erforderlich; eine wesentlich verbesserte Raum-Zeit-Ausbeute wird so erhalten. Alle diese vorteilhaften Ergebnisse sind im Hinblick auf den Stand der Technik überraschend. Man hätte insbesondere angesichts der höheren Reaktionstemperatur eine weit höhere Zersetzung des Reaktionsgemisches und die Bildung heterogener Gemische an Zersetzungs- und Nebenprodukten erwarten müssen.

Die Ausgangsstoffe II und III können in stöchiometrischer Menge oder im Überschuß jeder Komponente zur anderen, vorzugsweise in einem Verhältnis von 0,6 bis 1, insbesondere 0,9 bis 1 Mol Ausgangsstoff II je Hydroxygruppe und Mol Ausgangsstoff III, umgesetzt werden. Als Alkohole kommen Mono-, Di-, Tri-, Tetra-, Penta- und Hexaalkohole in Frage. Je nach dem entsprechenden Molverhältnis des Ausgangsstoffs II zum Ausgangsstoff III erhält man in diesem Falle Mono-, Di-, Tri-, Tetra-, Penta- und Hexa-hexahydropyrimidyl-4-äther bzw. bei einem Unterschuß, entsprechend den vorgenannten Mengenverhältnissen, an Ausgangsstoff II Mono-hydroxy-poly-hexahydropyrimidyl-4-äther bis Polyhydroxy-mono-hexahydropyrimidyl-4-äther. Bevorzugte Ausgangsstoffe II und III und dementsprechend bevorzugte Endstoffe I sind solche, in deren Formeln die einzelnen Reste $R^1$ und $R^2$ gleich oder verschieden sein können und jeweils einen Cyclohexylrest oder einen Aralkylrest mit 7 bis 12 Kohlenstoffatomen bedeuten, darüber hinaus auch die Reste $R^1$ jeweils

ein Wasserstoffatom, einen Phenylrest oder einen Alkylrest mit 1 bis 7 Kohlenstoffatomen bezeichnen können, $R^2$ auch für einen Alkylrest mit 1 bis 20, insbesondere 1 bis 7 Kohlenstoffatomen, der noch durch Di-(hydroxyäthyl)-aminogruppen, Phenoxygruppen, Cyclohexoxygruppen substituiert sein kann, einen Alkoxyalkylrest mit 3 bis 40, insbesondere 3 bis 14 Kohlenstoffatomen, wobei davon vorzugsweise 1 bis 20, insbesondere 1 bis 7 Kohlenstoffatome zu der Alkoxygruppe gehören, einen Alkyl- oder Alkenylrest mit 4 bis 20, insbesondere 4 bis 8 Kohlenstoffatomen, der durch die Gruppe

$$-\overset{\displaystyle O}{\overset{\|}{C}}-O-$$

unterbrochen ist, den Rest $R^5-O-(CH_2-CH_2-O)_w-(CH_2-CH_2)-$ , worin $R^5$ ein Wasserstoffatom oder einen Alkylrest mit 1 bis 14 Kohlenstoffatomen und w eine ganze Zahl, insbesondere 1 bis 14, bedeuten, einen Alkylenrest mit n Resten

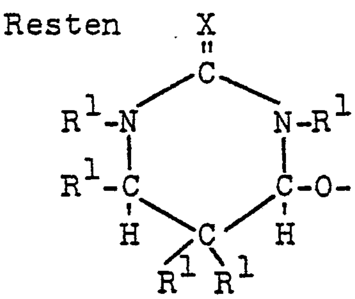

oder einen Alkylenrest mit n - y Resten

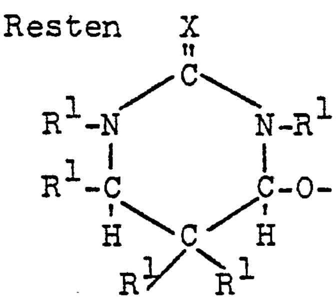

und y Hydroxygruppen und von n + 1 bis n + 12, insbesondere von n + 1 bis n + 6 Kohlenstoffatomen stehen kann, n 1, 4, 5 bezeichnet, y 0 oder eine ganze Zahl von 1 bis 5, X ein Sauerstoffatom oder ein Schwefelatom bedeutet, $R^3$ ein Wasserstoffatom bezeichnet, darüber hinaus auch, wenn $R^2$ für einen aliphatischen Rest mit mindestens 5 Kohlenstoffatomen

oder einen cycloaliphatischen oder araliphatischen Rest steht, auch eine Alkylgruppe mit 1 bis 3 Kohlenstoffatomen bedeuten kann, $R^4$ die Bedeutung von $R^2$ besitzt oder, wenn $R^2$ für die vorgenannten, bevorzugten Alkylenreste, die n oder n - y Reste

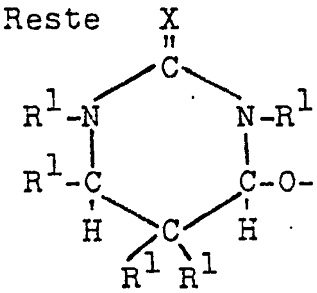

enthalten, steht, auch einen entsprechenden Alkylenrest mit n oder n + y Hydroxygruppen und von n + 1 bis n + 12, insbesondere von n + 1 bis n + 6 Kohlenstoffatomen stehen kann. Die vorgenannten Reste und Ringe können noch durch unter den Reaktionsbedingungen inerte Gruppen, z.B. Alkylgruppen mit 1 bis 4 Kohlenstoffatomen, substituiert sein.

So sind z.B. folgende Hexahydropyrimidine als Ausgangsstoffe II geeignet: 4-Hydroxy-2-oxo-hexahydropyrimidin, 5,5-Dimethyl-, 5,5-Diäthyl-, 5,5-Di-n-propyl-, 5,5-Diisopropyl-, 5,5-Di-n-butyl-, 5,5-Di-sek.-butyl-, 5,5-Diisobutyl-,5,5-Di-tert.-butyl-, 5-Phenyl-5-methyl-, 5-Benzyl-5-methyl-, 5-Cyclohexyl-5-methyl-, 5-Methyl-5-isopropyl-4-hydroxy-2-oxo-hexahydropyrimidin; entsprechende in 5-Stellung unsubstituierte, einfach oder zweifach durch vorgenannte Gruppen substituierte, in 1-, 3- und/oder 6-Stellung durch die Methyl-, Äthyl-, Hydroxyäthyl-, n-Propyl-, Isopropyl-, n-Butyl-, Isobutyl-, sek.-Butyl-, tert.-Butyl-, Cyclohexyl-, Benzyl-, Phenyl-gruppe substituierte 4-Hydroxy-2-oxo-hexahydropyrimidine; homologe 4-Methoxy-, 4-Äthoxy-, 4-n-Propoxy-, 4-Isopropoxy-2-oxo-hexahydropyrimidine; entsprechende, in vorgenannten Stellungen unsubstituierte und/oder substituierte 2-Thiono-hexahydropyrimidine.

Bevorzugte Ausgangsstoffe II sind:

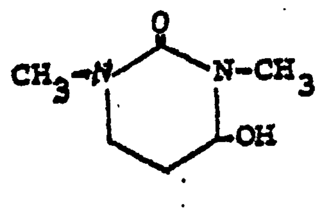

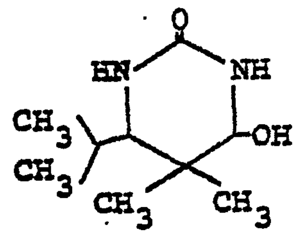

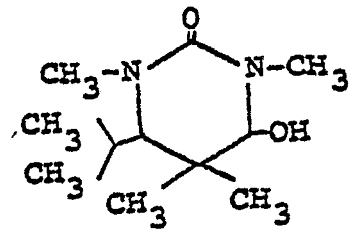

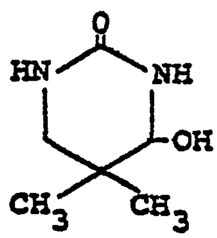

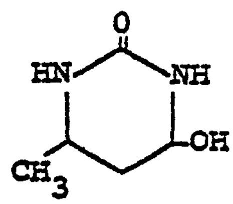

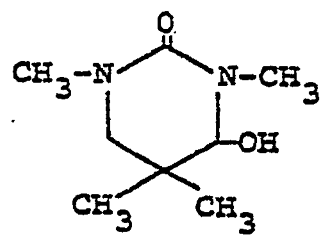

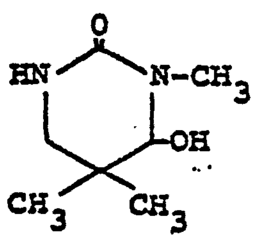

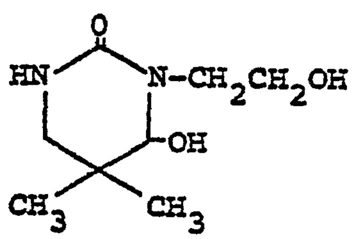

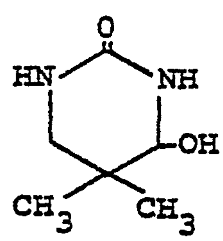

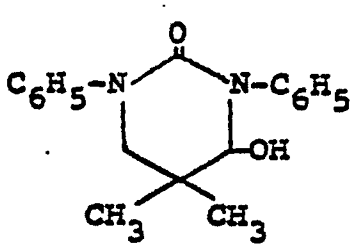

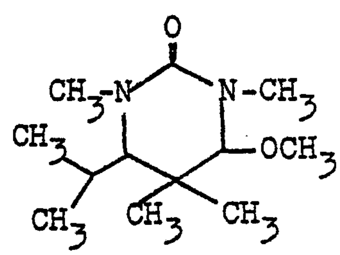

Es kommen z.B. als Alkohole III in Betracht: Undecanol, Dodecanol, Nonanol, n-Hexanol, 2-Äthylhexanol, Cyclohexanol, Benzylalkohol, Äthylenglykol; Diglykol, Triglykol, Tetraglykol, Pentaglykol, Hexaglykol, Heptaglykol, Octaglykol, Nonaglykol, Decaglykol, Undecaglykol, Dodecaglykol, Tridecaglykol, Tetradecaglykol, Propandiol-1,3, Butandiol-1,4, Propandiol-1,2, Neopentylglykol, 2,4-Pentylenglykol, 2,3-Butylenglykol, Hexandiol-1,6 und entsprechende Monoäther mit einer Alkylgruppe von 1 bis 18 Kohlenstoffatomen; Cyclopentanol, Cycloheptanol, Phenyläthylalkohol, n-Pentanol, Phenylpropanol, Cyclooctanol, n-Heptanol, n-Octanol, n-Decanol, Triäthanolamin; der Methyl-, Äthyl-, Propyl-, Isopropyl-, Butyl-, sek.-Butyl-, Isobutyl-, tert.-Butyl-monoäther des Äthylenglykols; Glycerin, Butantriole, Hexantriole, Pentaerythrit, 2-Methyl-2-methylol-propandiol-(1,3), 2-Hydroxymethyl-2-äthyl-propandiol-(1,3), Pentosen, Hexosen.

Bevorzugte Ausgangsstoffe III sind beispielsweise:

$CH_3OCH_2CH_2OH$ $HO-(CH_2CH_2O)_3H$ $HO(CH_2CH_2O)_{12}H$

$HOCH_2CH_2N(CH_2CH_2OH)_2$ $HOCH_2-CH_2OH$

$CH_3OCH_2CH_2OCH_2CH_2OH$ 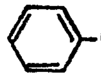$-OCH_2CH_2OH$

$C_{11}H_{23}OH$ $HOCH_2CH_2CH_2CH_2OCOCH = CH_2$

$C_9H_{19}OH$ $HOCH(CH_3)-CH_2OCOCH = CH_2$

$HOCH_2-CHOH-CHOH-CHOH-CHOH-CH_2OH$

$C_{13}H_{27}O(CH_2CH_2O)_{12}H$

Die Umsetzung wird bei einer Temperatur oberhalb von 110°C, zweckmäßig zwischen 110 und 180°C, vorteilhaft zwischen 120 und 170°C, vorzugsweise von 121 bis 160°C, insbesondere von 125 bis 155°C, drucklos oder unter Druck, kontinuierlich oder diskontinuierlich durchgeführt. Man kann ohne Lösungsmittel umsetzen; zweckmäßig verwendet man aber unter den Reaktionsbedingungen inerte Lösungsmittel, insbesondere mit Siedepunkten über 110°C. Bei Flüssigkeiten mit Siedepunkten unter 110°C muß die Reaktion in geschlossenen Gefäßen unter Druck durchgeführt werden. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Dimethylformamid und entsprechende Gemische. Bevorzugte Lösungsmittel sind Toluol und Xylole. Es ist nicht erforderlich, daß die Ausgangsstoffe und Endstoffe in diesen Lösungsmitteln löslich sind. Die Unlöslichkeit der Endstoffe ermöglicht eine besonders leichte Aufarbeitung. Ferner muß das gebildete Reaktionswasser nicht unbedingt abgeführt werden. Es ist jedoch vorteilhaft, das Wasser durch azeotrope Destillation abzutrennen. Zweckmäßig verwendet man das Lösungsmittel in einer Menge von 100 bis 10 000 Gewichtsprozent, vorzugsweise von 400 bis 1 000 Gewichtsprozent, bezogen auf Ausgangsstoff II. Die Umsetzung kann vorteilhaft in Abwesenheit von Lösungsmitteln durchgeführt werden, wenn eine oder beide Ausgangskomponenten oder der Endstoff flüssig oder bei der Reaktionstemperatur schmelzbar sind.

Die Reaktion wird schon aus Gründen des einfacheren bzw. wirtschaftlicheren Betriebs zweckmäßig ohne Zusatz von Säure durchgeführt. Gegebenenfalls kann man aber Säure, z.B. in Gestalt von Schwefelsäure, zweckmäßig in katalytischen Mengen, bevorzugt in Mengen von 0,005 bis 0,01 Äquivalenten Säure je Mol Ausgangsstoff II, oder auch Basen, z.B. in Gestalt von Natronlauge, zweckmäßig in katalytischen

Mengen, bevorzugt in Mengen von 0,005 bis 0,01 Äquivalenten Base je Ausgangsstoff II, zusetzen.

Die Reaktion kann wie folgt durchgeführt werden: Ein Gemisch der Ausgangsstoffe II und III und zweckmäßig Lösungsmittel wird während 0,25 bis 3 Stunden, insbesondere 0,25 bis 1 Stunde, bei der Reaktionstemperatur gehalten. Dann wird der Endstoff aus dem Gemisch in üblicher Weise, z.B. durch Kristallisation und Filtration oder Destillation, abgetrennt.

Die nach dem Verfahren der Erfindung herstellbaren Hexahydro-pyrimidyl-(4)-äther sind wertvolle Ausgangsstoffe für die Herstellung von Farbstoffen, Schädlingsbekämpfungsmitteln und Hilfsmitteln in der Textilchemie, Lackharzchemie, Holzwerkstoffindustrie. Ihre N-Methylol- und N-Alkoxymethylverbindungen sind als reaktive Vernetzer in der Textilchemie, Lackharzchemie, Holzwerkstoffindustrie vorteilhaft verwendbar.

Die in den Beispielen genannten Teile bedeuten Gewichtsteile.

Beispiel 1

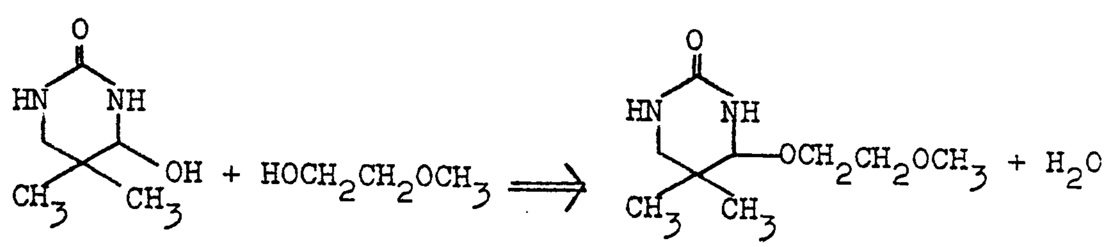

In einer Rührapparatur mit Rührer, Wasserabscheider, Rückflußkühler und Heizung werden 288 Teile 2-Oxo-4-hydroxy-5,5-dimethyl-hexahydropyrimidin zusammen mit 152 Teilen

Methylglykol und 600 Teilen Xylol eine halbe Stunde bei Rückflußtemperatur (132°C) erhitzt. Das entstehende Reaktionswasser wird aus dem Reaktionsgemisch durch azeotrope Destillation über den Wasserabscheider abgetrennt. Nach Abdestillieren des Xylols werden 398 Teile 2-Oxo-4-methoxyäthoxy-5,5-dimethyl-hexahydropyrimidin als kristallines Produkt erhalten. Das entspricht einer Ausbeute von 99 % der Theorie. Fp (Methylglykol) 112°C.

Beispiel 2

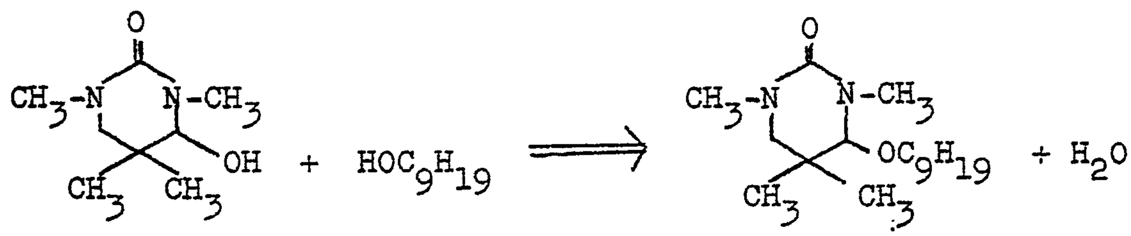

Analog Beispiel 1 werden 172 Teile 2-Oxo-4-hydroxy-1,3,5,5-tetramethyl-hexahydropyrimidin und 144 Teile n-Nonylalkohol in 500 Teilen Xylol 40 Minuten bei Rückflußtemperatur (130 bis 135°C) erhitzt, wobei das Reaktionswasser azeotrop über den Wasserabscheider abgetrennt wird. Nach Abdampfen des Xylols werden 288 Teile 2-Oxo- 4-nonoxy-1,3,5,5-tetramethyl-hexahydropyrimidin erhalten. Das entspricht einer Ausbeute von 96 % der Theorie. Siedepunkt 154 bis 158°C (0,2 mbar).

Beispiel 3

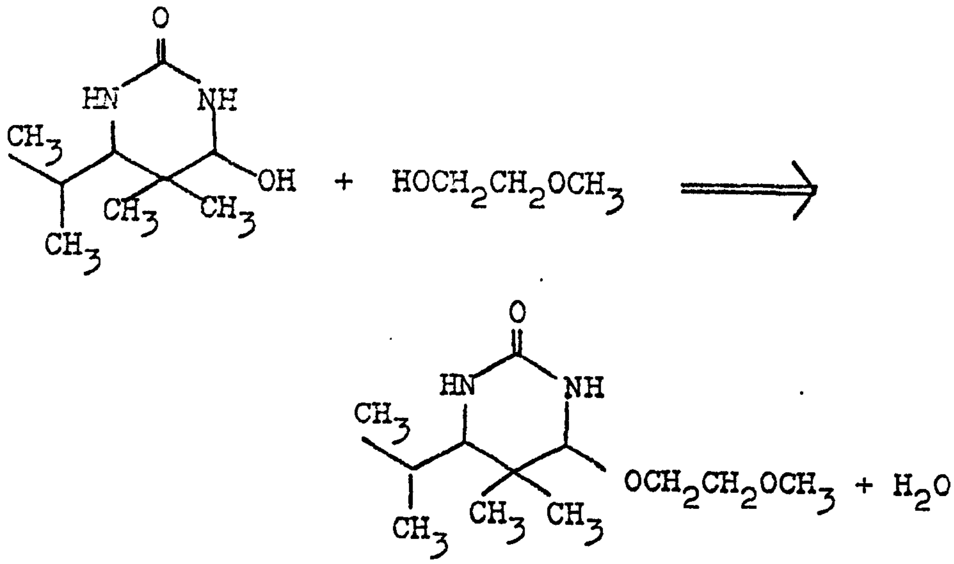

1 116 Teile 2-Oxo-4-hydroxy-5,5-dimethyl-6-isopropyl-hexahydropyrimidin werden analog Beispiel 1 mit 456 Teilen Methylglykol in 2 000 Teilen Xylol eine Stunde bei Rückflußtemperatur (130 bis 135°C) erwärmt. Das entstehende Reaktionswasser wird über einen Wasserabscheider abgetrennt (108 Teile). Nach Abdampfen des Xylols werden 1 408 Teile 2-Oxo-4-(methoxy-äthoxy)-5,5-dimethyl-6-isopropyl-hexahydropyrimidin in kristalliner Form erhalten. Das entspricht einer Ausbeute von 96 % der Theorie. Fp (Aceton) 112 bis 113°C.

Beispiel 4

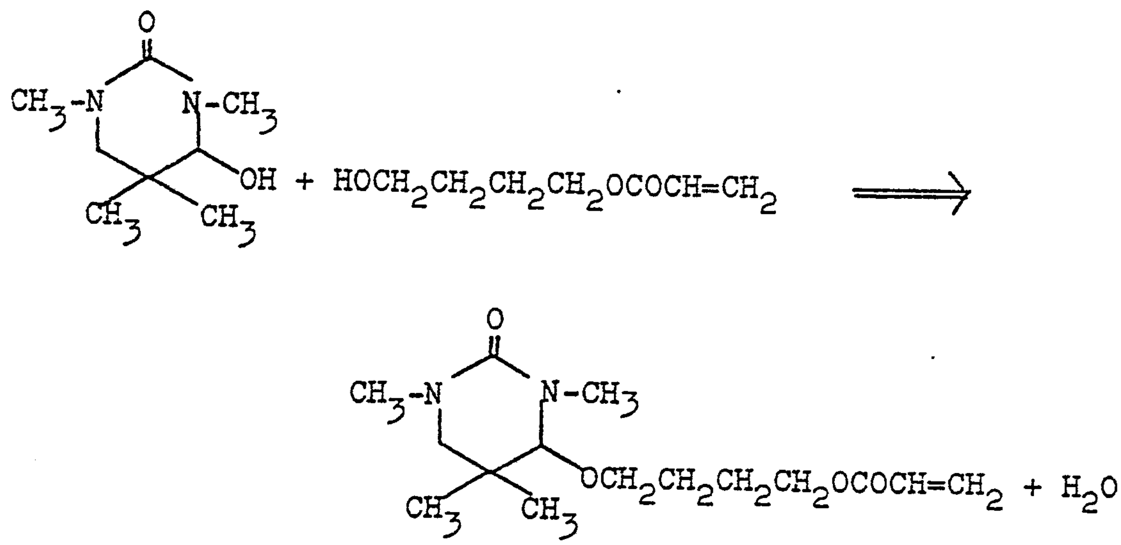

Analog Beispiel 1 wird das Gemisch aus 344 Teilen 2-Oxo-4-hydroxy-1,3,5,5-tetramethyl-hexahydropyrimidin und 288 Teilen Butandiol-monoacrylat in 1 000 Teilen Xylol eine Stunde auf Rückflußtemperatur (130 bis 135°C) unter Rühren erhitzt, wobei das Reaktionswasser über den Wasserabscheider abgetrennt wird. Es werden 36 Teile Wasser abgespalten. Nach Abdampfen des Xylols werden 576 Teile 2-Oxo-1,3,5,5-tetramethyl-pyrimidyl-(4)-äther des Butandiol-monoacrylat erhalten. Das entspricht einer Ausbeute von 97 % der Theorie. Siedepunkt 155 bis 158°C (0,2 bis 0,3 mbar).

Beispiel 5

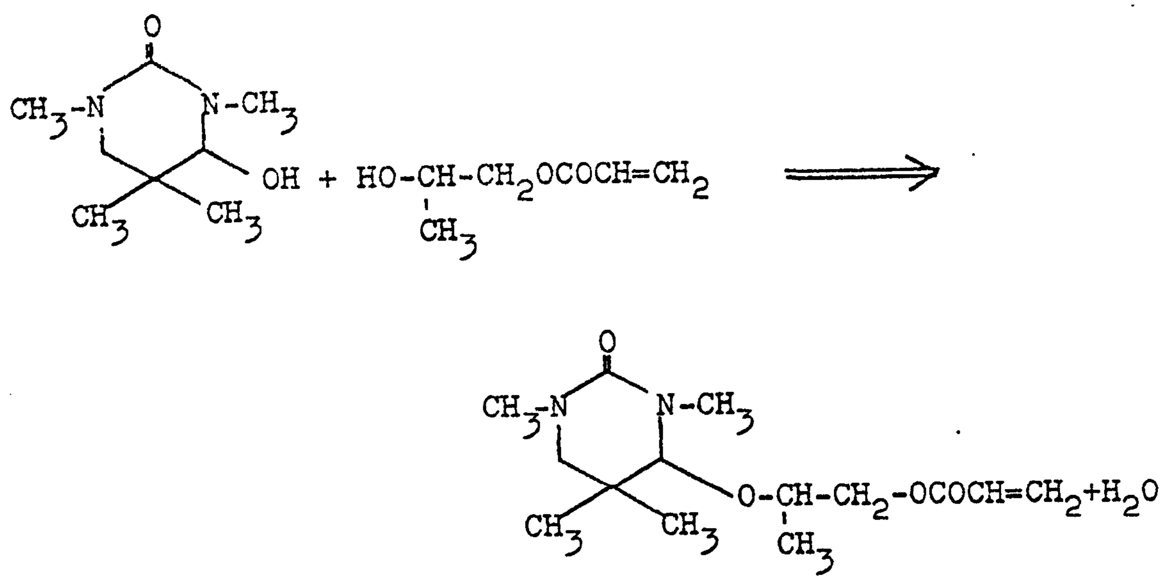

172 Teile 2-Oxo-4-hydroxy-1,3,5,5-tetramethyl-hexahydropyrimidin und 130 Teile 2-Hydroxy-isopropyl-acrylat werden analog Beispiel 1 in 500 Teilen Xylol eine Stunde bei Rückflußtemperatur (139°C) erhitzt. Das entstehende Reaktionswasser (18 Teile) wird über den Wasserabscheider abgetrennt. Nach Abdestillieren des Xylols werden 281 Teile 2-Oxo-1,3,5,5-tetramethyl-pyrimidyl-(4)-äther des 2-Hydroxy-isopropyl-acrylat erhalten. Das entspricht einer Ausbeute von 99 % der Theorie. Siedepunkt 136 bis 138°C (0,2 bis 0,4 mbar).

Beispiel 6

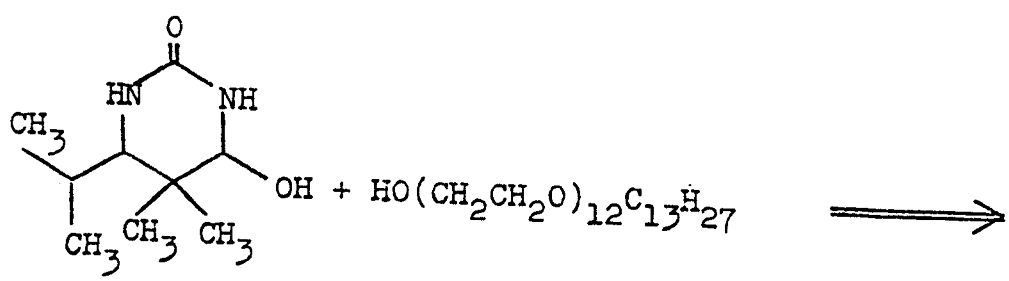

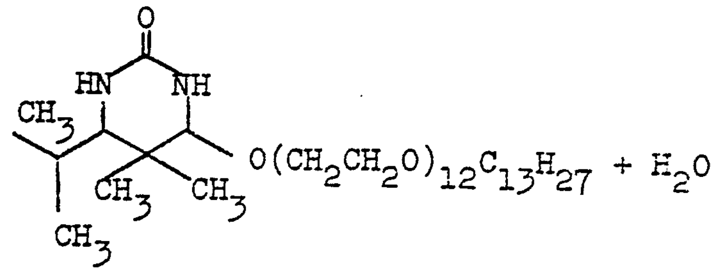

Analog Beispiel 1 wird das Gemisch aus 18,6 Teilen 2-Oxo-4-hydroxy-5,5-dimethyl-6-isopropyl-hexahydropyrimidin, 73 Teilen eines mit 12 Mol Äthylenoxid kondensierten Tridecylalkohols in 300 Teilen Xylol eine Stunde bei Rückflußtemperatur (130 bis 135°C) erwärmt, wobei das entstehende Reaktionswasser azeotrop abdestilliert wird. Nach Abdampfen des Xylols werden 88 Teile 2-Oxo-5,5,6,6-tetramethyl-pyrimidyl-(4)-äther des mit 12 Mol Äthylenoxid kondensierten Tridecylalkohols erhalten. Die Ausbeute beträgt 97 % der Theorie.

<u>Beispiel 7</u>

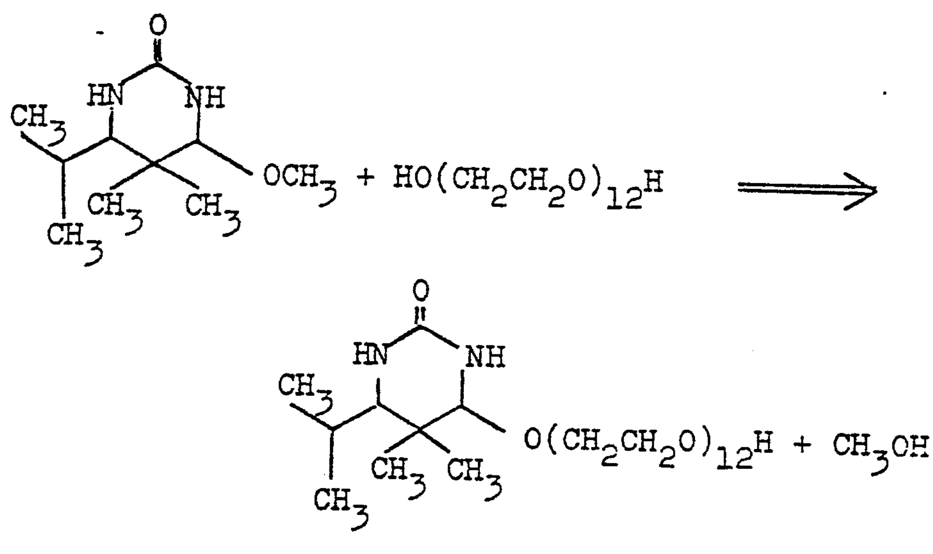

200 Teile 2-Oxo-4-methoxy-5,5-dimethyl-6-isopropyl-hexahydropyrimidin werden analog Beispiel 1 mit 546 Teilen Dodecaäthylenglykol eine Stunde bei 152°C unter Rühren erhitzt, wobei das abgespaltene Methanol abdestilliert wird. Es werden 702 Teile 2-Oxo-5,5,6,6-tetramethyl-pyrimidyl-(4)-äther des Dodecaäthylenglykols erhalten (98 % der Theorie).

Beispiel 8

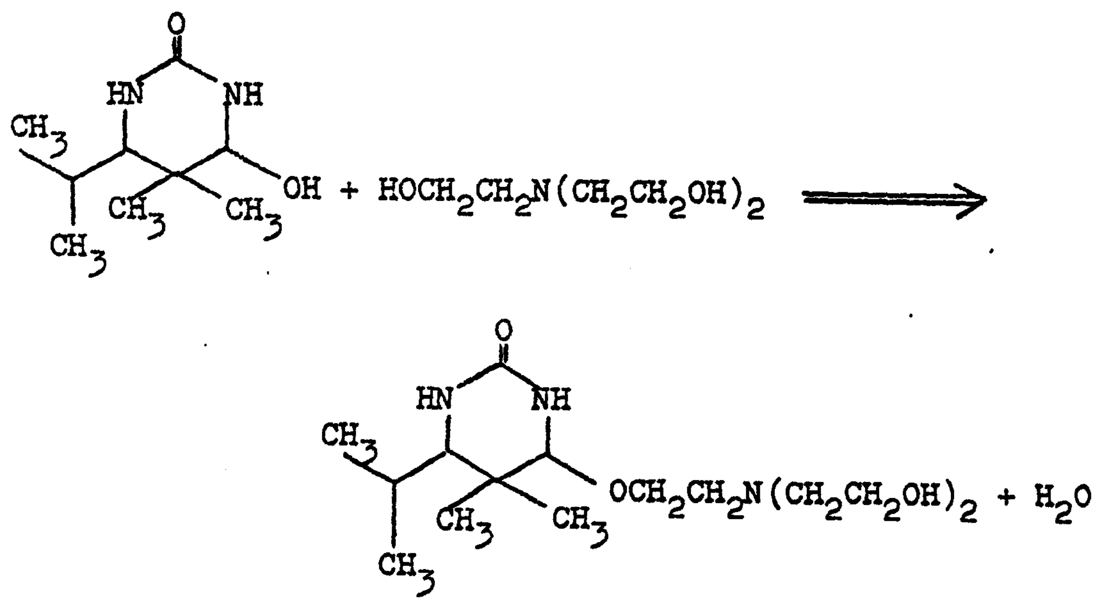

Analog Beispiel 1 werden 372 Teile 2-Oxo-4-hydroxy-5,5-dimethyl-6-isopropyl-hexahydropyrimidin mit 298 Teilen Triäthanolamin in 800 Teilen Xylol 2 Stunden bei Rückflußtemperatur (130 bis 135°C) erwärmt, wobei 36 Teile Wasser über den Wasserabscheider abgetrennt werden. Nach Abdampfen des Xylols werden 621 Teile 2-Oxo-5,5,6,6-tetramethyl-pyrimidyl-(4)-monoäther des Triäthanolamins erhalten. Das entspricht einer Ausbeute von 98 % der Theorie.

Beispiel 9

372 Teile 2-Oxo-4-hydroxy-5,5-dimethyl-6-isopropyl-hexahydropyrimidin werden analog Beispiel 1 mit 182 Teilen Sorbit in 800 Teilen Xylol 3 Stunden bei Rückflußtemperatur (137°C) erwärmt, wobei 36 Teile Wasser über einen Wasserabscheider abgetrennt werden. Nach Abdampfen des Xylols werden 511 Teile Sorbit, das anstelle von zwei Hydroxylgruppen durch zwei 2-Oxo-5,5-dimethyl-6-isopropyl-hexahydropyrimidin-4-oxy-Reste substituiert ist, erhalten. Der Endstoff zersetzt sich oberhalb von 220°C.

Beispiel 10 (Verwendung)

202 Teile des nach Beispiel 1 erhaltenen 2-Oxo-4-methoxyäthoxy-5,5-dimethyl-hexahydropyrimidins werden in einer Rührapparatur mit 150 Teilen einer 40-gewichtsprozentigen Formaldehydlösung versetzt und nach Einstellen eines pH-Wertes von 10 bis 11 mit Natronlauge bei 50°C unter Rühren 2 Stunden erwärmt. Nach Neutralisieren mit verdünnter Schwefelsäure wird durch Zusatz von Wasser eine 50-gewichtsprozentige Lösung hergestellt. Es werden 524 Teile einer 50-gewichtsprozentigen, wäßrigen Lösung von 2-Oxo-1,3-bishydroxymethyl-4-methoxyäthoxy-5,5-dimethyl-hexahydropyrimidin erhalten.
Ein gebleichtes und mercerisiertes Popeline-Baumwollgewebe mit einem Quadratmetergewicht von 125 g wird mit einer Lösung nachstehender Zusammensetzung durch Foulardieren imprägniert:
300 Teile einer 50-gewichtsprozentigen, wäßrigen Lösung von 2-Oxo-1,3-bishydroxymethyl-4-methoxyäthoxy-5,5-dimethylhexahydropyrimidin, 30 Teile einer 40-gewichtsprozentigen wäßrigen Dispersion eines Mischpolymerisates aus 89 Teilen Acrylsäure-n-butylester, 5 Teilen Butandioldiacrylat, 3 Teilen N-Methylolmethacrylamid und 3 Teilen Acrylamid,

1 Teil eines Adduktes aus 7 Teilen Äthylenoxid an 1 Mol i-Octylphenol, 30 Teilen einer wäßrig-alkoholischen Lösung eines oxäthylierten Polyamids und 20 Teile Magnesiumchloridhexahydrat werden mit Wasser auf 1 000 Raumteile verdünnt. Die Flottenaufnahme beträgt 70 bis 75 Prozent. Es wird auf einem Spannrahmen bei 100 bis 110°C getrocknet und anschließend auf dem Spannrahmen 5 Minuten bei 155°C erhitzt. Das ausgerüstete Gewebe zeigt folgende technologische Daten:

Trockenknitterwinkel nach DIN 53 890:

Kette + Schuß 278° (unbehandeltes Gewebe 65)

Naßknitterwinkel DIN 53 891:

Kette + Schuß 270° (unbehandeltes Gewebe 130)

Das Glättebild nach Monsanto weist eine Note von 3,5 bis 4 auf.

Die Werte nach 5 Maschinenkochwäschen betragen:

Trockenknitterwinkel nach DIN 53 890:

Kette + Schuß 256° (unbehandeltes Gewebe 76)

Naßknitterwinkel nach DIN 53 891:

Kette + Schuß 264° (unbehandeltes Gewebe 134)

Reißfestigkeit 40 x 100 mm Schuß 22 kg (unbehandeltes Gewebe 40)

Reißfestigkeit nach 5 Wäschen und 5 Chlorierungen 18 kg (unbehandeltes Gewebe 39)

Patentansprüche

1. Verfahren zur Herstellung von Hexahydropyrimidyl-(4)-äthern der Formel

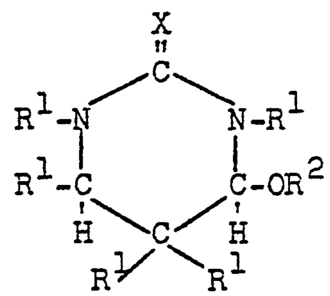

I,

worin die einzelnen Reste $R^1$ und $R^2$ gleich oder verschieden sein können und jeweils einen cycloaliphatischen oder araliphatischen Rest bedeuten, darüber hinaus auch die Reste $R^1$ jeweils ein Wasserstoffatom, einen aromatischen oder einen aliphatischen Rest bezeichnen können, $R^2$ auch für einen aliphatischen Rest, der darüber hinaus n Reste

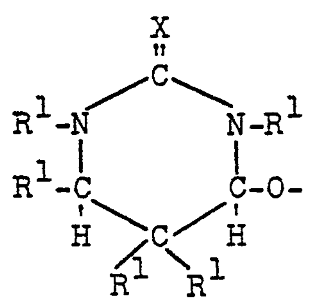

enthalten kann, stehen kann, n eine ganze Zahl von 1 bis 5 bezeichnet, X ein Sauerstoffatom oder ein Schwefelatom bedeutet, durch Umsetzung von 4-Hydroxy-hexahydropyrimidinen oder seinen Äthern mit Alkoholen, dadurch gekennzeichnet, daß man Hexahydropyrimidine der Formel

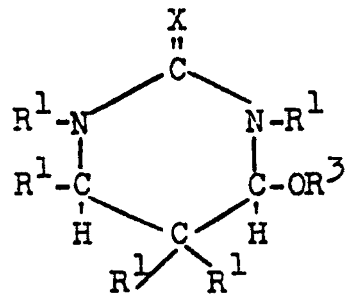

II,

worin $R^1$ und X die vorgenannte Bedeutung besitzen und $R^3$ ein Wasserstoffatom bezeichnet, darüber hinaus auch, wenn $R^2$ für einen aliphatischen Rest mit mindestens 5 Kohlenstoffatomen oder einen cycloaliphatischen oder araliphatischen Rest steht, auch eine Alkylgruppe mit 1 bis 3 Kohlenstoffatomen bedeuten kann, mit Alkoholen der Formel

$$R^4\text{-OH} \qquad \text{III,}$$

worin $R^4$ die Bedeutung von $R^2$ besitzt, oder, wenn $R^2$ für einen aliphatischen Rest mit n Resten

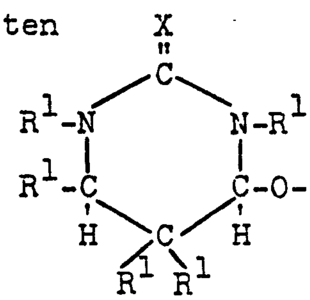

steht, auch denselben aliphatischen Rest mit anstelle der n Reste

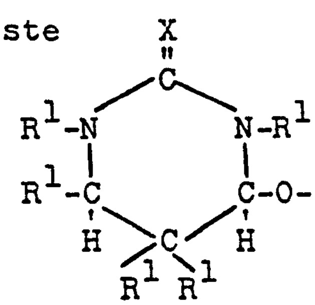

n Resten -OH bezeichnen kann, bei einer Temperatur oberhalb von 110°C umsetzt.

2. Polyhexahydropyrimidyl-(4)-äther der Formel

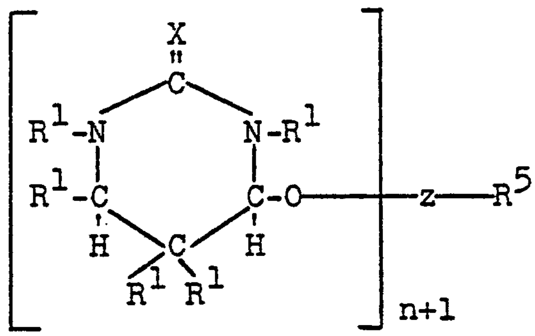

Ia,

worin die einzelnen Reste $R^1$ gleich oder verschieden sein können und jeweils ein Wasserstoffatom, einen aliphatischen, cycloaliphatischen, araliphatischen oder aromatischen Rest bedeuten, X ein Sauerstoffatom oder ein Schwefelatom bedeutet, n eine ganze Zahl von 1 bis 5 bedeutet, z eine n+1 entsprechende Zahl an Einfachbindungen bezeichnet, $R^5$ für einen aliphatischen Rest steht.

Europäisches Patentamt

# EUROPÄISCHER RECHERCHENBERICHT

Nummer der Anmeldung

EP 79 102 316.1

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - C - 1 231 247 (BADISCHE ANILIN- & SODA-FABRIK) * Anspruch * -- | 1 |
| | DE - A - 1 670 089 (BADISCHE ANILIN- & SODA-FABRIK) * Anspruch * -- | 1 |
| D | DE - A - 1 770 089 (BADISCHE ANILIN- & SODA-FABRIK) * Anspruch 1 * -- | |
| A | DE - A - 1 670 130 (BADISCHE ANILIN- & SODA-FABRIK) ---- | |

KLASSIFIKATION DER ANMELDUNG (Int.Cl.3)

C 07 D 239/10//
D 06 M 1/00

RECHERCHIERTE SACHGEBIETE (Int. Cl.3)

C 07 D 239/10

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 12-11-1979 | FROELICH |